# EUROPÄISCHE PATENTSCHRIFT

(11) **EP 3 221 287 B1**
(45) Veröffentlichungstag und Bekanntmachung des Hinweises auf die Patenterteilung: **02.01.2019**
(21) Anmeldenummer: 15794590.8
(22) Anmeldetag: 16.11.2015
(51) Int. Cl.: C07C 45/75, C07C 51/25, C07C 51/48

(54) **OPTIMIERTES VERFAHREN ZUR HERSTELLUNG VON METHACRYLSÄURE**
OPTIMIZED PROCESS FOR PREPARING METHACRYLIC ACID
PROCÉDÉ OPTIMISÉ POUR LA PRODUCTION D'ACIDE MÉTHACRYLIQUE

(30) Priorität: 19.11.2014 EP 14193871
(43) Veröffentlichungstag der Anmeldung: 27.09.2017
(73) Patentinhaber: Evonik Röhm GmbH, 64293 Darmstadt (DE)
(72) Erfinder: KRILL, Steffen, 64367 Mühltal (DE); BURGHARDT, Rudolf, 64287 Darmstadt (DE); RACZEK, Melanie, 64289 Darmstadt (DE); BALDUF, Torsten, 64319 Pfungstadt (DE)
(86) Internationale Anmeldenummer: PCT/EP2015/076667
(87) Internationale Veröffentlichungsnummer: WO 2016/079044

(56) Entgegenhaltungen:
- EP-A2- 0 194 620
- WO-A2-2012/154450

## Beschreibung

Die vorliegende Erfindung betrifft ein optimiertes Verfahren zur Herstellung von Methacrylsäure, wobei in einer ersten Stufe aus Propionaldehyd und Formaldehyd mittels einer Mannich-Reaktion Methacrolein hergestellt und dieses in einer zweiten Stufe zu Methacrylsäure oxidiert wird. Insbesondere betrifft die vorliegende Erfindung die Reduzierung der einzusetzenden Katalysatormengen in der ersten Stufe, insbesondere die Reduzierung der hier einzusetzenden Mengen an Säure durch die zusätzliche Installation von dazu geeigneten Recyclingströmen.

### Stand der Technik

Es besteht ein großes Interesse an möglichst einfachen, wirtschaftlichen und umweltschonenden Herstellungsverfahren für Methacrylsäure. Dabei ist insbesondere ein Verfahren zur Herstellung von Methacrylsäure auf Basis von C₂-Bausteinen von Interesse. Bei diesem Verfahren wird beispielsweise in einer Vorstufe aus Ethylen, Kohlenmonoxid und Wasserstoff Propionaldehyd hergestellt, welches anschließend über eine Mannich-ähnlichen Reaktion mit Formaldehyd zu Methacrolein umgesetzt wird. Diese Mannich-Reaktion wird dabei in der Regel mit einer Kombination aus Basen und Säuren katalysiert. In einer weiteren Stufe, im weiteren als Verfahrensschritt 2 bezeichnet, wird dann, z.B. in einer daran direkt angeschlossenen Anlage, dieses Methacrolein zu Methacrylsäure oxidiert. In weiteren Folgeschritten kann darauf z.B. die Veresterung zu Methylmethacrylat erfolgen.

Ein Problem dieser Methacrylsäure-Synthese ist, dass in der Mannich-Reaktion, im weiteren als Verfahrensschritt 1 bezeichnet, große Mengen Katalysator verbraucht werden. Daher besteht ein großes wirtschaftliches Interesse, die Menge einzusetzenden Katalysators zu verringern.

Das beschriebene C₂ -Verfahren zur Herstellung von Methacrolein, ist unter anderem in den Druckschriften US 7,141,702, US 4,408,079, JP 3069420, JP 4173757, EP 0 317 909 und US 2,848,499 nachzulesen.

Die zur Herstellung von Methacrolein geeigneten, auf einer Mannich-Reaktion basierenden Verfahren sind des weiteren Gegenstand entsprechender Übersichtsartikel, zum Beispiel in Ullmann's Encyclopedia of Industrial Chemistry 2012, Wiley-VCH Verlag GmbH & Co. KGaA, Weinheim, Acrolein and Methacrolein, DOI: 10.1002/14356007.a01_149.pub2.

In EP 0 194 620 ist eine entsprechende Kombination der Verfahrensschritte 1 und 2 offenbart, ohne dass auf die Katalysatorkonzentrationen in Verfahrensschritt 1 in irgendeiner Form eingegangen würde.

In DE 3213681 wird ein Verfahren zur Herstellung von MAL beschrieben, welches insbesondere dadurch charakterisiert ist, dass die Umsetzung bei einer Temperatur von größer 150 °C bei einer Reaktionszeit von höchstens 25 min in Gegenwart von sekundären Aminen und optional von Säuren durchgeführt wird. Dabei werden bezogen auf 1 mol Propionaldehyd zwischen 0,001 und 0,25, insbesondere zwischen 0,02 und 0,05 mol eines sekundären Amins als Base und zwischen 0 und 0,25 mol, insbesondere zwischen 0,02 und 0,05 mol einer Säure eingesetzt. Diese werden ausschließlich frisch dem Reaktor zugeführt und nach der Reaktion mit der wässrigen Phase von dem Methacrolein abgetrennt und entsorgt.

US 4,408,079 beschreibt ein Verfahren zur Herstellung von MAL, bei dem die Umsetzung von Propionaldehyd mit Formalin bei einem Molverhältnis von 0,9 bis 1,5 zu 1, einem pH-Wert zwischen 2,5 und 7 und Temperaturen von 0 °C bis 150 °C in Gegenwart von einem sekundären Amin, in einer Konzentration von 0,025 bis 0,75 bzw. von 0,05 bis 1,5 mol, und organischen Säuren in einer Konzentration von 0,05 bis 1,5 mol jeweils bezogen auf 1 mol Propionaldehyd durchgeführt wird. Auch die US 4,408,079 beschreibt ausschließlich die Frischzufuhr der Katalysatorkomponenten und die Abtrennung und Entsorgung nach der Reaktion. WO 2012/154450 offenbart ein Verfahren, bei dem mittels Oxidation aus Methacrolein Methacrylsäure hergestellt wird, wobei das Methacrolein aus Propylenglycol hergestellt wird. Alternativ kann das Methacrolein gemäss WO2012/154450 auch aus Formaldehyd und Propanal gewonnen werden, konkrete Details zu einem solchen Verfahren sind jedoch nicht offenbart.

### Aufgabe

In Anbetracht des Standes der Technik war es daher Aufgabe der vorliegenden Erfindung, ein Verfahren zur Herstellung von Methacrylsäure in Kombination mit der Synthese von Methacrolein (MAL) nach dem C₂-Verfahren in einem ersten Verfahrensschritt zur Verfügung zu stellen, bei dem gegenüber dem Stand der Technik nur eine insgesamt reduzierte Menge Katalysatoren in dem ersten Verfahrensschritt zugegeben werden muss.

Insbesondere bestand damit die Aufgabe, mit diesem Verfahren ein MAL als Vorstufe in der Synthese von Methacrylsäure herstellen zu können, welches unter Zugabe weniger frischer Säure, bei gegenüber dem Stand der Technik identischen Ausbeuten und Selektivitäten durchgeführt werden kann.

Weiterhin bestand die Aufgabe ein Verfahren zur Herstellung von Methacrylsäure, bestehend aus der Synthese von MAL mittels einer Mannich-Reaktion im ersten Verfahrensschritt und der anschließenden Oxidation von MAL zu Methacrylsäure in einem zweiten Verfahrensschritt, bei dem die Gesamtausbeute an Methacrylsäure gegenüber dem Stand der Technik gesteigert werden kann, zur Verfügung zu stellen. Weitere nicht explizit genannte Aufgaben ergeben sich aus dem Gesamtzusammenhang der nachfolgenden Beschreibung und der Ansprüche.

### Lösung

Gelöst werden die dieser Erfindung zugrunde liegenden Aufgaben mittels eines neuartigen Verfahrens zur kontinuierlichen Herstellung von Methacrylsäure, bei dem in einem ersten Verfahrensschritt aus Formaldehyd und Propionaldehyd mit mindestens einer Säure und mindestens einer organischen Base als Katalysatoren in einem Reaktor 1 Methacrolein hergestellt, anschließend von der dabei vorliegenden Katalysator-haltigen bzw. wässrigen Phase abgetrennt wird und in einem zweiten Verfahrensschritt mit einem heterogenen Katalysator in Gegenwart von Sauerstoff und Wasser in einem Reaktor 2 zu Methacrylsäure oxidiert wird. Der erste Verfahrensschritt stellt dabei eine Mannich-Reaktion dar.

Das in Verfahrensschritt 2 entstehende Prozessgas wird nach der Ableitung aus dem Reaktor 2 unter Erzeugung einer wässrigen Roh-Methacrylsäure kondensiert und gequencht. Anschließend erfolgt die Abtrennung der Methacrylsäure von der wässrigen Phase mit einem Extraktionsmittel. Bei diesem Extraktionsmittel handelt es sich in der Regel um ein organisches, nur geringfügig mit Wasser mischbares Lösungsmittel, welches einen geringen Siedepunkt aufweist. Beispiele dafür sind Alkane oder deren Mischung, wie zum Beispiel insbesondere Hexan oder Pentan.

Erfindungsgemäß wird die wässrige Phase von der organischen, die Methacrylsäure enthaltenden Phase nach der Extraktion ganz oder teilweise, direkt oder indirekt in den Reaktor 1 geleitet. Diese wässrige Phase enthält insbesondere Carbonsäuren, Diese Carbonsäuren wirken darauf in Reaktor 1 während der Mannich-Reaktion des Verfahrensschritts 1 als eine der beiden Katalysatorkomponenten und es muss - nach Einstellen des allgemeinen Reaktionsgleichgewichts - weniger oder keine frische Säure mehr in den Reaktor 1 geleitet werden.

Bei der organischen Base handelt es sich bevorzugt um ein sekundäres Amin, besonders bevorzugt um Dimethylamin. Bei der Säure, die dem Reaktor 1 zumindest beim Anfahren der kontinuierlichen Reaktion und zumeist auch im reduzierten Maße bei einer kontinuierlich durchgeführten Fahrweise frisch zugeleitet wird, kann es sich um eine organische oder eine anorganische Säure handeln. Bevorzugt handelt es sich um Schwefelsäure oder um eine organische Säure, insbesondere um, Ameisensäure, Essigsäure, Propionsäure und/oder Mischungen dieser Säuren, wobei auch Mischungen mehrerer Säuren eingesetzt werden können.

Bei den erfindungsgemäß aus Verfahrensschritt 2 direkt oder indirekt in den Reaktor 1 geleiteten, in der wässrigen Phase enthaltenen Carbonsäuren handelt es sich insbesondere um Essigsäure, Methacrylsäure, Propionsäure, Maleinsäure, Acrylsäure, Terephthalsäure. In der Regel handelt es sich insbesondere um Mischungen, mindestens eine dieser Säuren enthaltend, wobei in der Durchführung zumeist alle diese Säuren in der Mischung vorliegen und weitere Säuren, die als Nebenprodukt der Oxidation in Verfahrensschritt 2 gebildet werden oder als Nebenprodukt im MAL aus Verfahrensschritt 1 mit in den Reaktor 2 übertragen wurden. Dabei hängen die Verhältnisse der einzelnen Säuren zueinander insbesondere von den Prozessparametern des Verfahrensschritts 2 ab. So kann der Gehalt an Propionsäure oder Terephthalsäure insbesondere relativ klein sein oder diese in Konzentrationen an der Nachweisgrenze vorliegen. Methacrylsäure verbleibt dagegen bei der Extraktion in kleinen Mengen in der wässrigen Phase und liegt somit immer vor.

Charakteristisch ist es dabei, dass diese Säuren während der Oxidation des Methacroleins entstehen und als Mischung mit dem Produktwasser der Oxidation in Reaktor 1 geleitet werden.

Neben den genannten Säuren kann die in Reaktor 1 geleitete wässrige Phase auch weitere Säuren in sehr kleiner Konzentration enthalten. Beispiele hierfür sind Isophthalsäure, Benzoesäure, 4-Methylbenzoesäure oder das Oxidationsprodukt eines dimeren Methacroleins.

Des Weiteren kann das Abwasser Formaldehyd enthalten. Dieses Formaldehyd wird hauptsächlich in Reaktor 2 bei der Oxidation als Nebenprodukt gebildet. Der Gehalt der wässrigen Phase, die direkt oder indirekt dem Reaktor 1 zugeführt wird, kann dabei insbesondere zwischen 0,2 und 1,0 Gew% liegen. Formaldehyd stellt jedoch insbesondere ein Edukt der Methacroleinsynthese in Reaktor 1 dar. Damit ist ein weiterer überraschender Aspekt der vorliegenden Erfindung, dass nicht nur die Gesamteinsatzmenge der Katalysatorsäuren für die Reaktion in Reaktor 1 reduziert werden kann, sondern auch etwas weniger Formaldehyd in den Reaktor 1 frisch zugeführt werden muss, da ein Teil dieses Formaldehyds ebenfalls in Reaktor 1 zurückgeführt werden kann. Auch stellt eine weitere Zwischenaufbereitung des Recyclingstroms hier kein Problem dar, da Formaldehyd beispielsweise mittels einer Membran größtenteils zurückgehalten wird und damit im Recyclingstrom verbleibt.

Formaldehyd-haltige Abwässer haben darüber hinaus den großen Nachteil, dass dieser keiner biologischen Aufarbeitung zur Verfügung stehen. Damit stellt der Formaldehyd-Gehalt in dem Abwasser den primären Grund dafür dar, dass dieses einer Verbrennung zugeführt werden muss. Durch das erfindungsgemäße Verfahren mit einer ganzen oder teilweisen Rückführung der wässrigen Phase nach Verfahrensschritt 2 ist es jetzt möglich die Menge des in flüssiger Phase emittierten Formaldehyds deutlich zu reduzieren. Somit ist es möglich, das Gesamtverfahren mit deutlich geringeren Mengen, die einer Verbrennung zuzuführen sind, oder ganz ohne eine thermische Entsorgung durchzuführen.

Neben den geringeren Einsatzmengen an Säuren in Verfahrensschritt 1 ist damit ein weiterer Vorteil der vorliegenden Erfindung darin zu sehen, dass die in der wässrigen Phase der Extraktion, die an Verfahrensschritt 2 anschließt, verbleibende Methacrylsäure ganz oder zumindest teilweise in Reaktor 1 geleitet wird und von dort zusammen mit dem MAL zurück in Reaktor 2 übertragen wird. Somit steht diese nach Stand der Technik eigentlich verlorene Methacrylsäure einer erneuten Extraktion zur Verfügung und die Methacrylsäure-Ausbeute der Gesamtreaktion wird mit bis zu 1 Gew% gegenüber dem Stand der Technik gesteigert.

Insbesondere handelt es sich erfindungsgemäß bei mindestens 5 Gew%, bevorzugt mindestens 20 Gew% und besonders bevorzugt mindestens 50 Gew% der als Katalysator in den Reaktor 1 zugeleiteten Säure um die Carbonsäuren aus Verfahrensschritt 2. Bei einer Optimierung des Verfahrens kann es sogar möglich sein, Verfahrensschritt 1 nach dem Einstellen des Gleichgewichts vollständig ohne Zufuhr frischer Säure zu betreiben. Dieser Anteil recyclierter Säure gilt insbesondere für einen Zeitpunkt nach dem Erreichen eines stationären Zustands in dem kontinuierlichen Verfahren.

Das Einstellen des Gleichgewichts bedeutet erfindungsgemäß, dass bei der kontinuierlich betriebenen Reaktion nach dem Anfahren der Reaktion ein Zustand in den Verfahrensschritten 1 und 2 erreicht wird, in dem die Innentemperaturen und der Innendruck der Reaktoren sowie die Zuleitungs-, Recycling-, Überführungs- und Abfuhrströme der beiden Reaktionsschritte nur noch sehr geringen Schwankungen, von maximal 5 °C, 2 bar bzw. 5% unterliegen. Unter Zuleitungsströmen werden dabei Ströme verstanden, mit denen Reaktanten, Lösungsmittel, wie Wasser, Katalysatoren oder weitere Hilfsstoffe Reaktor 1, Reaktor 2 oder einer Kolonne in einem der Verfahrensschritte frisch zugeleitet werden.

Unter Recyclingströmen werden Ströme verstanden, mit denen Reaktionsphasen zurück in einen vorgeschalteten Reaktor 1 oder 2 geleitet werden. Dabei kann es sich neben dem erfindungsgemäßen Strom, enthaltend die wässrige Lösung der Carbonsäuren, aus Verfahrensschritt 2 in Reaktor 1 auch beispielsweise um die zumindest teilweise Zurückleitung des Sumpfes einer nachgeschalteten Kolonne in Reaktor 1 handeln. Bei Überführungsströmen handelt es sich dagegen um Ströme, die dem eigentlichen Reaktionsverlauf folgen. Beispiele dafür sind der Ablauf aus Reaktor 1 in eine nachgeschaltete Kolonne oder der Zulauf des MAL's in Reaktor 2. Bei den Abfuhrströmen wiederum handelt es sich um Ströme, mittels derer Phasen endgültig aus der Anlage entfernt werden. Neben der Produktentnahme kann es sich dabei zum Beispiel auch um ein Abgas oder die Entnahme einer z.B. wässrigen Phase zur Entsorgung handeln.

In einer besonderen Ausführungsform der vorliegenden Erfindung wird die wässrige Phase aus Verfahrensschritt 2 vor der Einleitung in den Reaktor 1 mittels einer Destillation oder einer Membrantrennstufe aufkonzentriert. Diese Ausführung hat den großen Vorteil, dass einerseits zwar größere Mengen der Säure in Reaktor 1 geleitet werden können, dabei jedoch die Menge des mit überführten Wassers eher gering ist.

Bei einer entsprechenden Destillation können die säurereichen Phasen sowohl im verbleibenden Sumpf, als auch im Kopf der Kolonne oder über einen oder mehrere Seitenströme abgeführt werden. Es ist auch möglich die säurehaltigen Phasen an mehreren dieser Stellen der Kolonne zu entnehmen und danach wieder zu vereinigen, bevor sie in Reaktor 1 geleitet werden.

Bevorzugt und gleichzeitig unabhängig von der sonstigen Ausführungsform des erfindungsgemäßen Verfahrens wird die umgesetzte Reaktionslösung des Verfahrensschritts 1 nach der Entnahme am Austritt des Reaktors in einer Kolonne destilliert. Anschließend wird die der Kolonne entnommene Methacrolein-haltige Phase in einem Phasentrenngefäß in Methacrolein und eine wässrige Phase getrennt. Diese wässrige Phase kann darauf ganz oder teilweise zurück in die Kolonne geführt werden. In der Regel ist diesem Phasentrenner ein Kondensator zur Verflüssigung des Kopfstroms aus der Destillationskolonne vorgeschaltet.

In einer genauso bevorzugten alternativen Ausführungsform der Erfindung wird die umgesetzte Reaktionslösung des Verfahrensschritts 1 nach der Entnahme am Austritt des Reaktors in einer Kolonne destilliert. In dieser Ausführungsform wird darauf der Kopfstrom dieser Destillation anschließend direkt in Reaktor 2 geleitet, ohne dass eine zwischenzeitliche Phasentrennung vorgenommen wird.

Unabhängig von der Ausgestaltung mit oder ohne anschließende Phasentrennung wird besonders bevorzugt ein Teil, insbesondere mindestens 20 Gew% der wässrigen Phase aus dem Sumpf der Kolonne, enthaltend Säuren und organische Basen, sowie den jeweiligen Basenanteil in den Salzen mit den Säuren und in den basenhaltigen Zwischenprodukten des Verfahrensschritts 1, in den Eintritt des Reaktors 1 recycliert. Dieser Strom wird im weiteren als Recyclingstrom bezeichnet.

In einer weiteren, gleichfalls bevorzugten Ausführungsform des erfindungsgemäßen Verfahrens wird die wässrige Phase aus Verfahrensschritt 2 ganz oder teilweise in diese, Verfahrensschritt 1 nachgeschaltete Kolonne geleitet, und von dort zumindest teilweise zusammen mit dem Sumpf der Kolonne, dem zuvor beschriebenen Recyclingstrom, in den Reaktor 1 geleitet. Optional kann der Sumpf aus der Kolonne vor der Einleitung in den Reaktor 1 mittels einer weiteren Destillation oder einer Membrantrennstufe aufkonzentriert werden. Diese Aufarbeitung kann dabei unabhängig davon, ob die wässrige Phase aus Verfahrensschritt 2 in diesen Sumpf geleitet wird oder nicht, installiert sein.

Alternativ dazu, und genauso bevorzugt wird die wässrige Phase aus Verfahrensschritt 2 ganz oder teilweise direkt in Reaktor 1 geleitet wird. Für diesen Fall ist es möglich, wenn auch weniger bevorzugt, für diesen Strom und den Recyclingstrom aus dem Kolonnensumpf zwei getrennte Membrantrennstufen und/oder Destillationen zu installieren.

Mehr bevorzugt ist eine Ausführungsform des vorliegenden Verfahrens, bei der die wässrige Phase aus Verfahrensschritt 2 nach der Extraktion und vor der Einleitung in den Reaktor 1 mit mindestens einem Teil des Sumpfes aus der dem Reaktor 1 nachgeschalteten Kolonne, also dem Recyclingstrom, gemischt wird und die Mischung optional mittels einer Destillation oder einer Membrantrennstufe aufkonzentriert wird, bevor dieser kombinierte Strom in Reaktor 1 geleitet wird.

In einer solchen Membrantrennstufe können beispielsweise Membranen der Nanofiltration oder Umkehrosmose, die zur Abtrennung von Wasser aus einer Mischung geeignet sind, verwendet werden. Dazu bekannt sind Membranen aus dem Bereich der Wasseraufarbeitung bzw. Wasserentsalzung, wie sie sonst zum Beispiel zur Gewinnung von Trinkwasser oder Kesselspeisewasser in Kraftwerken Verwendung finden. Bevorzugt handelt es sich um Membranen, die eine trennaktive Schicht aus Polyamid, Celluloseacetat oder Polyethersulfon, besonders bevorzugt aus einem Polyamid aufweisen. Ein gut geeignetes Beispiel dafür ist die Dow Filmtec SW30HR Membran.

In der Regel liegen die Membranen als Spiralwickelelemente vor. Der genaue Aufbau solcher Membranen kann beispielsweise in Th. Melin, R. Rautenbach "Membranverfahren - Grundlagen der Modul- und Anlagenauslegung", 3. Auflage, Springer Verlag, Berlin, S. 173-175 nachgelesen werden.

Die Membrantrennstufe kann dabei beispielsweise bei einer lokalen Temperatur an der Membran zwischen 10 und 70 °C, bevorzugt zwischen 30 und 40 °C betrieben werden. Der Transmembrandruck liegt dabei beispielsweise zwischen 20 und 100 bar, bevorzugt zwischen 70 und 90 bar. Die genaue Anlagentechnik zur Inkludierung der Membrantrennstufen in eine solche Produktionsanlage ist dem Fachmann bekannt und kann beispielsweise in Th. Melin, R. Rautenbach "Membranverfahren - Grundlagen der Modul- und Anlagenauslegung", 3. Auflage, Springer Verlag, Berlin, S. 205-226 und 245-308 nachgelesen werden.

Das erfindungsgemäße Verfahren umfasst in Verfahrensschritt 1 die Herstellung von Methacrolein durch Umsetzung von Propanal mit Formaldehyd über eine Aldol- bzw. Mannichkondensation. Das Formaldehyd kann dabei beispielsweise als Formalin eingesetzt werden. Die hierzu geeigneten Verfahren sind dem Fachmann bekannt und Gegenstand entsprechender Übersichtsartikel, zum Beispiel in Ullmann's Encyclopedia of Industrial Chemistry 2012, Wiley-VCH Verlag GmbH & Co. KGaA, Weinheim, Acrolein and Methacrolein, DOI: 10.1002/14356007.a01_149.pub2. Insbesondere sei auch auf die besonders bevorzugte Ausführung dieses Verfahrensschritts 1, wie sie in WO 2014/170223 beschrieben ist, verwiesen.

Bevorzugt wird die Umsetzung in Verfahrensschritt 1 bei einer Temperatur von 100 bis 300 °C, einer Verweilzeit der Reaktionsmischung in Reaktor 1 zwischen 1 und 30 s, besonders bevorzugt zwischen 5 und 15 s, und bei einem Druck von 5 bis 100 bar durchgeführt. Bevorzugt weist der Zulauf in Reaktor 1 ein Verhältnis von Propionaldehyd zu Formaldehyd zwischen 1 zu 1,2 mol und 1 zu 0,8 mol auf. Insgesamt enthält der Zulauf in Reaktor 1 einschließlich der wässrigen Phase aus Verfahrensschritt 2 und dem optionalen Recyclingstrom aus der Kolonne bezogen auf ein mol organische Base bevorzugt zwischen 1 und 3 mol, besonders bevorzugt zwischen 1 und 1,5 mol Säure. Diese Angaben beziehen sich naturgemäß auf die Säureäquivalente und nicht auf die Stoffäquivalente. Dies muss bei der Verwendung von di-Säuren, wie insbesondere Schwefelsäure, berücksichtigt werden.

Bevorzugt ist der Wassergehalt im Gesamtzulauf des Reaktors 1 größer 50 Gew% und maximal 85 Gew% ist. Genauso bevorzugt ist die Menge an organischer Base, bevorzugt einem sekundären Amin, beispielsweise Dimethylamin, im Zulauf des Reaktors, umfassend die reine Base, sowie den jeweiligen Basenanteil in den Salzen mit den Säuren und in den basenhaltigen Zwischenprodukten des Verfahrensschritts 1 bezogen auf Propionaldehyd 0,1 bis 20 Mol%, bevorzugt mehr als 2 Mol%, besonders bevorzugt mehr als 5 Mol%. Entsprechend wird die Reaktion dann mit 0,1 bis 20 Mol%, bevorzugt mehr als 2 Mol%, besonders bevorzugt mindestestens 5 mol% Säure oder den entsprechenden Säureanteilen in den Salzen mit der Base durchgeführt..

Insbesondere stammen bevorzugt mindestens 20 % der Gesamtmenge organischer Base des Gesamtzulaufs aus einem Frisch-Feed. Der Rest wird dann mittels des Recyclingstroms aus dem Kolonnensumpf zugeleitet.

In der Regel werden die Verfahrensparameter des Verfahrensschritts 1 derart eingestellt, dass die Methacroleinkonzentration der Reaktionsmischung am Ablauf des Reaktors 1 zwischen 20 und 45 Gew% liegt.

Bei der Herstellung von Methacrolein aus Propanal und Formaldehyd wird das Reaktionsgemisch - wie beschrieben - anschließend einer Kolonne zugeleitet und dort bevorzugt mit Wasserdampf gestrippt. Das Methacrolein verlässt zusammen mit Wasser die Kolonne am Kopf. Das Gemisch wird kondensiert und bevorzugt über einen Phasentrenner, insbesondere ein Phasentrenngefäß, in eine obere und eine untere Phase getrennt. Die obere Phase enthält das Methacrolein und wird direkt oder über optionale weitere Reinigungsvorrichtungen in den Reaktor gemäß Verfahrensschritt 2 weitergeleitet. Die untere Phase besteht hauptsächlich aus Wasser. Vorzugsweise wird diese - wie bereits beschrieben - zur Entfernung des noch darin gelösten Methacroleins zumindest teilweise wieder in die Kolonne zurückgeführt.

In der Regel befindet sich zwischen der Destillationskolonne und dem Phasentrenner noch ein Kondensator.

Der Wassergehalt des Roh-Methacroleins aus der Destillation kann temperaturbedingt variieren. Vorzugsweise wird die nach der Umsetzung von Formaldehyd mit Propanal erhaltene Reaktionsmischung demgemäß auf eine Temperatur abgekühlt, bei der sich der Wassergehalt in der Methacroleinphase einstellt. Vorzugsweise kann die Temperatur im Phasentrenner zwischen 0 und 50 °C, vorzugsweise 5 bis 30 °C und besonders bevorzugt 10 bis 25 °C eingestellt werden.

Die wässrige Katalysatorlösung kann am Sumpf der Kolonne zusammen mit dem bei der Reaktion gebildeten Wasser und dem Wasser aus der eingesetzten Formaldehydlösung abgezogen werden. Für die Weiterverarbeitung kann die Sumpfflüssigkeit teilweise oder ganz, batchweise oder kontinuierlich verworfen werden. Insbesondere ist es möglich, den Sumpfablauf so in zwei Teilströme aufzuteilen, dass ein Teilstrom gerade die Menge an Wasser mit sich führt, die bei der Reaktion gebildet und mit den Ausgangsstoffen eingegeben wurde. Dieser Teilstrom wird dann ausgeschleust und der restliche Anteil in den Reaktor zurückgeführt. Wässriges Formaldehyd und Propanal können auch getrennt vorgeheizt und dem Reaktor zugefahren werden.

### Beispiele:

Eine Formalinlösung mit einem Formaldehydgehalt von 37 Gew% und Propionaldehyd wurden vermischt (im Weiteren als Aldehydlösung bezeichnet) und anschließend in einem ölbeheizten Wärmetauscher auf die gewünschte Temperatur (siehe Tab.1) erwärmt. Eine Katalysatorlösung, die Essigsäure (bzw. Retentat oder Raffinat oder Mischungen) und Dimethylamin (als 40 Gew%ige Lösung in Wasser) enthielt, wurde ebenfalls auf die gewünschte Temperatur von 160 °C vorgewärmt. Die vorgewärmte Aldehydlösung und die vorgewärmte Katalysatorlösung wurden darauf in einem statischen Mischer vermischt. Diese Eduktmischung wurde dann einem mittels Öl temperierten Rohrreaktor (1/8" Wendel, 6 m; 1,44 id, 10,1 mL Reaktorvolumen) zugeführt. Die Reaktion wurde bei einem Druck von 55 bar durchgeführt. Die Verweilzeit im Reaktor betrug zwischen 9,4 bis 9,7 s. Die Produktmischung am Ablauf des Rohrreaktors wurde über ein Ventil entspannt und gelangte in die Produktkolonne zur Destillation. Am Kopf dieser Kolonne wurde nach Kondensation und Phasentrennung ein zweiphasiges Gemisch aus Methacrolein und einer wässrigen Phase erhalten. Die Analyse dieses Gemischs erfolgte mittels GC-Analytik (Varian CP 3800, Säule: DB Wax, Detektoren: WLD und FID). Weitere Analytik erfolgte mittels HPLC-Analytik, Gerät: Agilent 1200, Säulen: Agilent SB-Aq, UV-Detektor.

### Beispiel 1:

Für die Katalysatorlösung wurde anstatt Essigsäure (Vergleichsbeispiele) das Retentat einer zweistufigen Membrananlage verwendet. Dazu wurde die nach Verfahrensschritt 2 erhaltene Quenchliquid extrahiert und das so erhaltene Abwasser (Zusammensetzung bestimmt mittels HPLC-Analytik: 2,7 Gew% Essigsäure; 0,1 Gew% Ameisensäure; 0,2 Gew% Maleinsäure; 0,007 Gew% Acrylsäure; 0,6 Gew% Methacrylsäure; 0,02 Gew% Phthalsäure; 0,03 Gew% Isophthalsäure; 0,004 Gew% Methylmethacrylat; 0,02 Gew% Benzoesäure; 0,005 Gew% DiMAL-Säure (Oxidationsprodukt des dimeren Methacroleins); 0,02 Gew% Terephthalsäure) in einer zweistufigen Membrananlage (Membran 1 Stufe, Duplex NF, Filmtec SW30H, Modul SuS251L ; Membran 2 Stufe, PCS-NF, Filmtech SW 30, Flachmembran 240 cm²) aufkonzentriert. Als Frischfeed wurden 0,8 - 0,9 kg/h Abwasser dosiert. Hierbei wurde eine Temperatur von 30 °C gewählt und ein Druck von 80 bar für die erste Stufe gewählt. Die 2 Stufe wurde ebenfalls bei 30 °C, jedoch bei 50 bar betrieben. Das mittels Membran erhaltene Retentat hatte folgende Zusammensetzung: 6,4 Gew% Essigsäure; 0,5 Gew% Ameisensäure; 0,6 Gew% Maleinsäure; 0,21 Gew% Acrylsäure; 1,5 Gew% Methacrylsäure; 0,05 Gew% Phthalsäure; 0,07 Gew% Isophthalsäure; 0,009 Gew% Methylmethacrylat; 0,05 Gew% Benzoesäure; 0,01 Gew% DiMAL-Säure (Oxidationsprodukt des dimeren Methacroleins); 0,05 Gew% Terephthalsäure (bestimmt mittels HPLC-Analytik). Hierbei wurden die verschiedenen Säuren als Essigsäureäquivalente betrachtet.

Dieses Retentat wurde mit DMA gemischt und als Katalysatorlösung nach der beschriebenen Vorgehensweise verwendet. Die Bedingungen und Ausbeuten sind in Tabelle 1 aufgeführt.

Überraschend wurde festgestellt, dass sich das Retentat als Ersatz der Essigsäure eignet. Hierdurch konnten hohe Ausbeuten erzielt werden.

### Beispiel 2:

In einem weiteren Versuch wurde eine Mischung aus Retentat (Zusammensetzung siehe Beispiel 1) und reiner Essigsäure eingesetzt. Die Einwaagen wurden so gewählt, dass die Essigsäure zu 40 mol% Säureäquivalent durch das Retentat ersetzt wurde. Die Versuchsbedingungen wurden wie im vorhergehenden Beispiel gewählt und der Versuch analog durchgeführt.

### Beispiel 3:

Hier wurde das Raffinat der Extraktion für die hergestellte Katalysatorlösung verwendet. Das Abwasser, das nach der Extraktion anfällt, wurde für diesen Versuch nicht behandelt und direkt eingesetzt. Die Versuchsbedingungen wurden analog den vorherigen Beispielen eingestellt und der Versuch analog durchgeführt.

### Beispiel 4:

Schließlich wurde die Quenchliquid als Katalysatorersatz eingesetzt. Diese enthält noch einen hohen Anteil an Methacrylsäure. Die genaue Zusammensetzung der verwendeten Quenchliquid war die folgende:
2,7 Gew% Essigsäure; 0,15 Gew% Ameisensäure; 0,26 Gew% Maleinsäure; 0,14 Gew% Acrylsäure; 33,0 Gew% Methacrylsäure; 0,02 Gew% Phthalsäure; 0,03 Gew% Isophthalsäure; 0,01 Gew% Methylmethacrylat; 0,07 Gew% Benzoesäure; 0,01 Gew% DiMAL-Säure; 0,05 Gew% Terephthalsäure; 0,009 Gew% 4-Methylbenzoesäure (bestimmt mittels HPLC-Analytik).

Die Versuchsbedingungen wurden wie im vorhergehenden Beispiel gewählt und der Versuch analog durchgeführt.

### Vergleichsbeispiel 1:

Die Katalysatorlösung wurde mit reiner Essigsäure (Eisessig) angesetzt. Die Versuchsbedingungen wurden wie bei den Beispielen 1 bis 4 gewählt und der Versuch analog durchgeführt.

### Vergleichsbeispiel 2:

Die Katalysatorlösung wurde mit reiner Essigsäure (Eisessig) angesetzt. Die Versuchsbedingungen wurden wie bei den Beispielen 1 bis 4 gewählt. Anstatt einer 1/8"-Kapillare kam ein Plattenwärmetaucher der Firma IMM mit gleichem Reaktorvolumen (10,1 mL) zum Einsatz.

Eine weitere Ausbeuteerhöhung kann angenommen werden, da sich Metharcylsäure in Methacrolein nachweisen lassen konnte. Dieses wird der Oxidation zugeführt und trägt dort zu einer Erhöhung der Gesamtausbeute bei.

**Tabelle 1: Bedingungen der Methacroleinsynthese; Umsatz und Selektivität**

| | **Propionaldehyd/ Formaldehyd** | **Dimethylamin/ Propionaldehyd** | **Dimethylamin/ Essigsäure (Äquivalente)** | **Reaktionstemperatur** | **Umsatz (Propionaldehyd)** | **Selektivität (Methacrolein)** | **Ausbeute** |
|---|---|---|---|---|---|---|---|
| | [mol/mol] | [mol/mol] | [mol/mol] | [°C] | [%] | [%] | [%] |
| **Beispiel 1** | 1,00 | 0,054 | 0,91 | 160 | 98,9 | 98,5 | 97,4 |
| **Beispiel 2** | 1,00 | 0,054 | 0,89 | 160 | 99,6 | 98,4 | 98,0 |
| **Beispiel 3** | 1,00 | 0,053 | 0,91 | 160 | 99,0 | 98,5 | 97,5 |
| **Beispiel 4** | 1,00 | 0,056 | 0,89 | 160 | 99,8 | 98,5 | 98,3 |
| | | | | | | | |
| **Vergleichsbeispiel 1** | 1,00 | 0,052 | 0,90 | 160 | 99,7 | 98,2 | 98,0 |
| **Vergleichsbeispiel 2** | 1,00 | 0,042 | 0,91 | 160 | 99,5 | 97,4 | 96,9 |

## Patentansprüche

1. Verfahren zur kontinuierlichen Herstellung von Methacrylsäure, bei dem in einem ersten Verfahrensschritt aus Formaldehyd und Propionaldehyd mit mindestens einer Säure und mindestens einer organischen Base als Katalysatoren in einem Reaktor 1 Methacrolein hergestellt, anschließend dieses Methacrolein von der dabei vorliegenden Katalysator-haltigen Phase abgetrennt wird und in einem zweiten Verfahrensschritt mit einem heterogenen Katalysator in Gegenwart von Sauerstoff und Wasser in einem Reaktor 2 zu Methacrylsäure oxidiert wird, wonach das dabei entstehende Prozessgas unter Erzeugung einer wässrigen Roh-Methacrylsäure kondensiert und gequencht wird und anschließend mit einem Extraktionsmittel von der wässrigen Phase abgetrennt wird, **dadurch gekennzeichnet, dass** die wässrige Phase nach der Extraktion, enthaltend Carbonsäuren, ganz oder teilweise, direkt oder indirekt in den Reaktor 1 geleitet wird.

2. Verfahren gemäß Anspruch 1, **dadurch gekennzeichnet, dass** es sich bei der organischen Base um ein sekundäres Amin, bevorzugt um Dimethylamin handelt, und dass es sich bei der Säure, die dem Reaktor 1 frisch zugeleitet wird, um Schwefelsäure, Ameisensäure, Essigsäure, Propionsäure und/oder Mischungen dieser Säuren handelt.

3. Verfahren gemäß Anspruch 1 oder 2, **dadurch gekennzeichnet, dass** es sich bei den Carbonsäuren, die in der aus dem zweiten Verfahrensschritt in den Reaktor 1 geleiteten wässrigen Phase enthalten sind, um Essigsäure, Methacrylsäure, Propionsäure, Maleinsäure, Acrylsäure, Terephthalsäure oder um Mischungen, mindestens eine dieser Säuren enthaltend, handelt.

4. Verfahren gemäß mindestens einem der Ansprüche 1 bis 3, **dadurch gekennzeichnet, dass** es sich bei mindestens 5 Gew%, bevorzugt mindestens 50 Gew% der als Katalysator in den Reaktor 1 zugeleiteten Säure um die Carbonsäuren aus Verfahrensschritt 2 handelt.

5. Verfahren gemäß mindestens einem der Ansprüche 1 bis 4, **dadurch gekennzeichnet, dass** die wässrige Phase aus Verfahrensschritt 2 nach der Extraktion und vor der Einleitung in den Reaktor 1 mittels einer Destillation oder einer Membrantrennstufe aufkonzentriert wird.

6. Verfahren gemäß mindestens einem der Ansprüche 1 bis 5, **dadurch gekennzeichnet, dass** die umgesetzte Reaktionslösung des Verfahrensschritts 1 nach der Entnahme am Austritt des Reaktors in einer Kolonne destilliert wird und anschließend das Methacrolein in einem Phasentrenngefäß von der sich abscheidenden wässrigen Phase getrennt wird, wobei diese wässrige Phase ganz oder teilweise zurück in die Kolonne geführt wird.

7. Verfahren gemäß mindestens einem der Ansprüche 1 bis 5, **dadurch gekennzeichnet, dass** die umgesetzte Reaktionslösung des Verfahrensschritts 1 nach der Entnahme am Austritt des Reaktors in einer Kolonne destilliert wird und der Kopfstrom dieser Destillation anschließend direkt in Reaktor 2 geleitet wird.

8. Verfahren gemäß Anspruch 6 oder 7, **dadurch gekennzeichnet, dass** mindestens 20 Gew% der wässrigen Phase aus dem Sumpf der Kolonne, enthaltend Säuren und organische Basen, sowie den jeweiligen Basenanteil in den Salzen mit den Säuren und in den basenhaltigen Zwischenprodukten des Verfahrensschritts 1, in den Eintritt des Reaktors 1 recycliert werden.

9. Verfahren gemäß mindestens einem der Ansprüche 1 bis 8, **dadurch gekennzeichnet, dass** die wässrige Phase aus Verfahrensschritt 2 direkt in Reaktor 1 geleitet wird.

10. Verfahren gemäß Anspruch 8, **dadurch gekennzeichnet, dass** die wässrige Phase aus Verfahrensschritt 2 in die Kolonne gemäß Anspruch 6 bzw. 7 und von dort zumindest teilweise zusammen mit dem Sumpf der Kolonne in den Reaktor 1 geleitet wird.

11. Verfahren gemäß Anspruch 10, **dadurch gekennzeichnet, dass** der Sumpf aus der Kolonne vor der Einleitung in den Reaktor 1 mittels einer Destillation oder einer Membrantrennstufe aufkonzentriert wird.

12. Verfahren gemäß mindestens einem der Ansprüche 1 bis 11, **dadurch gekennzeichnet, dass** die wässrige Phase aus Verfahrensschritt 2 und vor der Einleitung in den Reaktor 1 mit mindestens einem Teil des Sumpfes gemäß Anspruch 7 gemischt wird und die Mischung optional mittels einer Destillation oder einer Membrantrennstufe aufkonzentriert wird.

13. Verfahren gemäß mindestens einem der Ansprüche 1 bis 12, **dadurch gekennzeichnet, dass** der Zulauf in Reaktor 1 ein Verhältnis von Propionaldehyd zu Formaldehyd zwischen 1 zu 1,2 mol und 1 zu 0,8 mol aufweist und einschließlich der wässrigen Phase aus Verfahrensschritt 2 und dem optionalen Recyclingstrom aus der Kolonne bezogen auf ein mol organische Base zwischen 1 und 3 mol Säure enthält.

14. Verfahren gemäß mindestens einem der Ansprüche 1 bis 13, **dadurch gekennzeichnet, dass** der Wassergehalt im Gesamtzulauf des Reaktors 1 größer 50 Gew% und maximal 85 Gew% ist, die Menge an organischer Base im Zulauf des Reaktors, umfassend die reine Base, sowie den jeweiligen Basenanteil in den Salzen mit den Säuren und in den basenhaltigen Zwischenprodukten des Verfahrensschritts 1 bezogen auf Propionaldehyd mehr als 2 Mol% beträgt und die Verweilzeit der Reaktionsmischung in Reaktor 1 zwischen 1 und 30 s liegt.

## Claims

1. Process for continuously preparing methacrylic acid, in which methacrolein is prepared in a first process step from formaldehyde and propionaldehyde with at least one acid and at least one organic base as catalysts in a reactor 1, then this methacrolein is separated from the catalyst-containing phase present and oxidized in a second process step to methacrylic acid with a heterogeneous catalyst in the presence of oxygen and water in a reactor 2, and then the process gas formed is condensed and quenched to produce an aqueous crude methacrylic acid and then separated from the aqueous phase with an extractant, **characterized in that** the aqueous phase after being extracted, comprising carboxylic acids, is passed wholly or partly, directly or indirectly, into reactor 1.

2. Process according to Claim 1, **characterized in that** the organic base is a secondary amine, preferably dimethylamine, and the acid which is fed fresh to reactor 1 is sulphuric acid, formic acid, acetic acid, propionic acid and/or mixtures of these acids.

3. Process according to Claim 1 or 2, **characterized in that** the carboxylic acids present in the aqueous phase passed from the second process step into reactor 1 are acetic acid, methacrylic acid, propionic acid, maleic acid, acrylic acid, terephthalic acid or mixtures comprising at least one of these acids.

4. Process according to at least one of Claims 1 to 3, **characterized in that** at least 5% by weight, preferably at least 50% by weight, of the acid fed into reactor 1 as catalyst is the carboxylic acids from process step 2.

5. Process according to at least one of Claims 1 to 4, **characterized in that** the aqueous phase from process step 2 after being extracted and before being introduced into reactor 1 is concentrated by means of a distillation or a membrane separation stage.

6. Process according to at least one of Claims 1 to 5, **characterized in that** the converted reaction solution from process step 1 after being withdrawn at the reactor exit is distilled in a column and then the methacrolein is separated in a phase separation vessel from the aqueous phase that separates out, said aqueous phase being returned wholly or partly to the column.

7. Process according to at least one of Claims 1 to 5, **characterized in that** the converted reaction solution from process step 1 after being withdrawn at the reactor exit is distilled in a column, and the top stream from this distillation is subsequently passed directly into reactor 2.

8. Process according to Claim 6 or 7, **characterized in that** at least 20% by weight of the aqueous phase is recycled from the column bottoms comprising acids and organic bases and the particular proportion of base in the salts with the acids and in the base-containing intermediates of process step 1 is recycled into the inlet of reactor 1.

9. Process according to at least one of Claims 1 to 8, **characterized in that** the aqueous phase from process step 2 is passed directly into reactor 1.

10. Process according to Claim 8, **characterized in that** the aqueous phase from process step 2 is passed into the column according to Claim 6 or 7 and thence at least partly into reactor 1 together with the column bottoms.

11. Process according to Claim 10, **characterized in that** the column bottoms before being introduced into reactor 1 are concentrated by means of a distillation or a membrane separation stage.

12. Process according to at least one of Claims 1 to 11, **characterized in that** the aqueous phase from process step 2, before being introduced into reactor 1, is mixed with at least a portion of the bottoms according to Claim 7 and the mixture is optionally concentrated by means of a distillation or a membrane separation stage.

13. Process according to at least one of Claims 1 to 12, **characterized in that** the feed into reactor 1 has a ratio of propionaldehyde to formaldehyde between 1:1.2 mol and 1:0.8 mol and, including the aqueous phase from process step 2 and the optional recycling stream from the column, contains between 1 and 3 mol of acid based on one mole of organic base.

14. Process according to at least one of Claims 1 to 13, **characterized in that** the water content in the overall feed to reactor 1 is greater than 50% by weight and not more than 85% by weight, the amount of organic base in the feed to the reactor, comprising the pure base and the particular proportion of base in the salts with the acids and in the base-containing intermediates of process step 1, based on propionaldehyde, is more than 2 mol% and the residence time of the reaction mixture in reactor 1 is between 1 and 30 s.

## Revendications

1. Procédé de fabrication continue d'acide méthacrylique, selon lequel, lors d'une première étape de procédé, de la méthacroléine est fabriquée à partir de formaldéhyde et de propionaldéhyde avec au moins un acide et au moins une base organique en tant que catalyseurs dans un réacteur 1, puis cette méthacroléine est séparée de la phase contenant des catalyseurs présente et oxydée lors d'une deuxième étape de procédé avec un catalyseur hétérogène en présence d'oxygène et d'eau dans un réacteur 2 pour former de l'acide méthacrylique, le gaz de procédé ainsi formé étant condensé et désactivé avec formation d'un acide méthacrylique brut aqueux, puis séparé de la phase aqueuse avec un agent d'extraction, **caractérisé en ce que** la phase aqueuse après l'extraction, contenant des acides carboxyliques, est conduite directement ou indirectement, en totalité ou en partie, dans le réacteur 1.

2. Procédé selon la revendication 1, **caractérisé en ce que** la base organique consiste en une amine secondaire, de préférence la diméthylamine, et **en ce que** l'acide qui est introduit sous forme fraîche dans le réacteur 1 consiste en de l'acide sulfurique, de l'acide formique, de l'acide acétique, de l'acide propionique et/ou des mélanges de ces acides.

3. Procédé selon la revendication 1 ou 2, **caractérisé en ce que** les acides carboxyliques qui sont contenus dans la phase aqueuse introduite dans le réacteur 1 à partir de la deuxième étape de procédé consistent en de l'acide acétique, de l'acide méthacrylique, de l'acide propionique, de l'acide maléique, de l'acide acrylique, de l'acide téréphtalique ou des mélanges contenant au moins un de ces acides.

4. Procédé selon au moins l'une quelconque des revendications 1 à 3, **caractérisé en ce qu'**au moins 5 % en poids, de préférence au moins 50 % en poids, de l'acide introduit dans le réacteur 1 en tant que catalyseur consiste en les acides carboxyliques de l'étape de procédé 2.

5. Procédé selon au moins l'une quelconque des revendications 1 à 4, **caractérisé en ce que** la phase aqueuse de l'étape de procédé 2 est concentrée au moyen d'une distillation ou d'une étape de séparation sur membrane après l'extraction et avant l'introduction dans le réacteur 1.

6. Procédé selon au moins l'une quelconque des revendications 1 à 5, **caractérisé en ce que** la solution réactionnelle non réagie de l'étape de procédé 1 est distillée dans une colonne après le soutirage à la sortie du réacteur, puis la méthacroléine est séparée dans un récipient de séparation de phases de la phase aqueuse qui se sépare, cette phase aqueuse étant recyclée en totalité ou en partie dans la colonne.

7. Procédé selon au moins l'une quelconque des revendications 1 à 5, **caractérisé en ce que** la solution réactionnelle non réagie de l'étape de procédé 1 est distillée dans une colonne après le soutirage à la sortie du réacteur, puis le courant de tête de cette distillation est introduit directement dans le réacteur 2.

8. Procédé selon la revendication 6 ou 7, **caractérisé en ce qu'**au moins 20 % en poids de la phase aqueuse issue du fond de la colonne, contenant des acides et des bases organiques, ainsi que la proportion de bases respective dans les sels avec les acides et dans les produits intermédiaires contenant des bases de l'étape de procédé 1, est recyclée dans l'entrée du réacteur 1.

9. Procédé selon au moins l'une quelconque des revendications 1 à 8, **caractérisé en ce que** la phase aqueuse de l'étape de procédé 2 est introduite directement dans le réacteur 1.

10. Procédé selon la revendication 8, **caractérisé en ce que** la phase aqueuse de l'étape de procédé 2 est introduite dans la colonne selon la revendication 6 ou 7, et est introduite depuis celle-ci au moins en partie conjointement avec le fond de la colonne dans le réacteur 1.

11. Procédé selon la revendication 10, **caractérisé en ce que** le fond de la colonne est concentré avant l'introduction dans le réacteur 1 au moyen d'une distillation ou d'une étape de séparation sur membrane.

12. Procédé selon au moins l'une quelconque des revendications 1 à 11, **caractérisé en ce que** la phase aqueuse de l'étape de procédé 2 est mélangée avant l'introduction dans le réacteur 1 avec au moins une partie du fond selon la revendication 7, et le mélange est éventuellement concentré au moyen d'une distillation ou d'une étape de séparation sur membrane.

13. Procédé selon au moins l'une quelconque des revendications 1 à 12, **caractérisé en ce que** l'alimentation dans le réacteur 1 présente un rapport entre le propionaldéhyde et le formaldéhyde compris entre 1 sur 1,2 mole et 1 sur 0,8 mole, et contient entre 1 et 3 moles d'acide par rapport à une mole de base organique, y compris la phase aqueuse issue de l'étape de procédé 2 et le courant de recyclage éventuel issu de la colonne.

14. Procédé selon au moins l'une quelconque des revendications 1 à 13, **caractérisé en ce que** la teneur en eau dans l'alimentation totale du réacteur 1 est supérieure à 50 % en poids et est d'au plus 85 % en poids, la quantité de base organique dans l'alimentation du réacteur, comprenant la base pure, ainsi que la proportion de bases respective dans les sels avec les acides et dans les produits intermédiaires contenant des bases de l'étape de procédé 1, par rapport au propionaldéhyde, est supérieure à 2 % en moles, et le temps de séjour du mélange réactionnel dans le réacteur 1 est compris entre 1 et 30 s.
